# FASCICULE DE BREVET EUROPEEN

(11) **EP 1 049 443 B1**
(45) Date de publication et mention de la délivrance du brevet: **21.04.2004**
(21) Numéro de dépôt: 99936737.8
(22) Date de dépôt: 16.08.1999
(51) Int. Cl.: A61K 7/00, A61K 7/06

(54) **COMPOSITION CAPILLAIRE CONTENANT UN POLYCONDENSAT COMPRENANT AU MOINS UN MOTIF POLYURETHANNE ET/OU POLYUREE ET UN POLYOL**
Zusammensetzung für die Haarbehandlung, die ein Polykondensat mit mindestens einer Polyurethan- und/oder Polyharnstoffeinheit und ein Polyol enthält.
HAIR COMPOSITION CONTAINING A POLYCONDENSATE COMPRISING AT LEAST A POLYURETHANE AND/OR POLYUREA UNIT AND A POLYOL

(30) Priorité: 27.08.1998 FR 9810782
(43) Date de publication de la demande: 08.11.2000
(73) Titulaire: L'OREAL, 75008 Paris (FR)
(72) Inventeur: STURLA, Jean-Michel, F-92100 Boulogne-Billancourt (FR); BREMENSON, Jean-Luc, F-75019 Paris (FR)
(74) Mandataire: Bourdeau, Françoise
(86) Numéro de dépôt international: PCT/FR1999/001991
(87) Numéro de publication internationale: WO 2000/012051

(56) Documents cités:
- WO-A-97/15275
- DE-A- 4 241 118
- DE-A- 19 541 326
- US-A- 5 626 840
- DATABASE WPI Week 9351 Derwent Publications Ltd., London, GB; AN 93-410762 XP002106178 "Foam-type aerosol hairdressing material without choking of spraying outlet - comprises base agent, e.g. polyhydric alcohol, foaming agent, 1-3 carbon lower alcohol, spraying agent and water" & JP 05 310535 A (LION & OSAKA AEROSOL KOGYO), 22 novembre 1993 (1993-11-22)

## Description

L'invention a pour objet des compositions capillaires conditionnées sous forme aérosol et comprenant dans un milieu cosmétiquement acceptable, un polymère multiséquencé comprenant au moins un motif polyuréthanne et/ou polyurée, au moins un polyol, un solvant organique et un gaz propulseur. Elle vise également un procédé pour la mise en forme ou le maintien de la coiffure comprenant la mise en oeuvre de ces compositions ainsi que leur utilisation pour la fabrication de produits capillaires, en vue d'obtenir un maintien ou une mise en forme de la coiffure.

La fixation de la coiffure est un élément important du coiffage qui consiste à maintenir la mise en forme déjà réalisée ou à mettre en forme les cheveux et à les fixer simultanément.

Les produits capillaires pour la mise en forme et/ou le maintien de la coiffure les plus répandus sur le marché de la cosmétique sont des compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution peut être conditionnée par exemple dans un récipient aérosol approprié mis sous pression à l'aide d'un propulseur.

La qualité de la pulvérisation obtenue au moyen d'un dispositif aérosol, c'est-à-dire essentiellement la distribution des gouttelettes dans l'espace à la sortie de la buse, dépend fortement de la constitution chimique de la composition mise en oeuvre. On porte donc un intérêt tout particulier à la formulation de compositions cosmétiques qui donnent lieu à une qualité de pulvérisation de plus en plus satisfaisante.

Les compositions destinées à la fixation et/ou au maintien de la coiffure présentent parfois l'inconvénient d'altérer les propriétés cosmétiques des cheveux.

Ainsi, les cheveux peuvent devenir rêches et perdre leur douceur naturelle. On recherche donc des compositions de coiffage qui fixent et/ou maintiennent bien la coiffure tout en procurant de bonnes propriétés cosmétiques.

Il est connu par le brevet DE 195 41 326 des compositions de coiffage distribuées à partir d'un dispositif aérosol qui contiennent, dans un milieu hydroal-coolique, un polymère à motif polyuréthanne en tant que polymère fixant, et un propulseur. Ces compositions, qui donnent déjà satisfaction en terme de fixation de la coiffure, peuvent toutefois être améliorées en ce qui concerne notamment les propriétés cosmétiques qu'elles confèrent aux cheveux tout en offrant une qualité de pulvérisation optimale.

De manière surprenante et inattendue, la Demanderesse a découvert, contre toute attente, qu'en associant certains agents de conditionnement particuliers à un polycondensat contenant au moins un motif polyuréthanne et/ou polyurée, il est possible de satisfaire aux exigences mentionnées ci-dessus.

L'invention a pour objet une composition capillaire destinée à être appliquée à partir d'un dispositif aérosol comprenant, dans un milieu cosmétiquement acceptable, en proportion relative en poids par rapport au poids total de la composition:
(i) 0,1 à 20 % d'un polycondensat comprenant au moins une séquence po lyuréthanne et/ou polyurée,
(ii) 7,5 à 70% d'un solvant organique,
(iii) 15 à 85 % d'un gaz propulseur,
caractérisée par le fait que la composition comprend en outre de 0,01 à 20 % d'au moins un polyol ou d'un mélange de ceux-ci, le rapport en poids du gaz propulseur au solvant organique étant supérieur ou égal à 1,75.

Au sens de la présente invention, on entend par "polyol", un composé en C₂ à C₁₄ de type hydrocarbure aliphatique linéaire, ramifié ou cyclique, saturé ou insaturé, portant au moins deux fonctions hydroxyles sur la chaîne alkyle, ainsi que les polymères de type polyéther de ces composés alkyles polyhydroxylés.

Un autre objet de l'invention concerne un procédé de mise en forme ou de maintien de la coiffure comprenant la mise en oeuvre de cette composition.

Encore un autre objet de l'invention concerne l'utilisation de cette composition pour la fabrication de compositions capillaires, en vue d'obtenir un maintien
ou une mise en forme de la coiffure.

Les polycondensats comprenant au moins une séquence polyuréthanne et/ou polyurée particulièrement visés par la présente invention sont ceux décrits dans les brevets EP 0 751 162, EP 0 637 600, FR 2 743 297 et EP 0 648 485 dont la Demanderesse est Titulaire, ainsi que les brevets EP 0 656 021 ou WO 94/ 03510 de la Sociéré Basf ou EP 0 619 111 de la Société National Starch.

Les polycondensats utilisés conformément à l'invention peuvent être solubles dans le milieu cosmétiquement acceptable, notamment après neutralisation par une base organique ou minérale, ou encore former une dispersion dans ce milieu. La dispersion peut comprendre alors au moins 0,05 % de tensioactif permettant la mise en dispersion et le maintien en dispersion du polycondensat.

Selon l'invention, on peut utiliser tout type de tensioactif dans ladite dispérsion, mais de préférence un tensioactif non ionique. La taille moyenne des particules du polycondensat dans la dispersion est de préférence comprise entre 0,1 et 1 micron.

A titre d'exemple, le polycondensat peut être formé par un arrangement de blocs, cet arrangement étant obtenu notamment à partir de:
(1) au moins un composé qui contient deux ou plus de deux atomes d'hydro gène actifs par molécule;
(2) au moins un diol ou un mélange de diols contenant des radicaux acides
   ou leurs sels;
(3) au moins un di- ou polyisocyanate.

Avantageusement, les composés (1) sont choisis dans le groupe comprenant les diols, les diamines, les polyesterols, les polyétherols ou leur mélange.

Les composés (1) qui sont préférés sont les polyéthylène et les polypropylène glycols linéaires, en particulier ceux qui sont obtenus par réaction de l'oxyde d'éthylène ou de propylène avec l'eau ou du diéthylène ou du dipropylène glycol en présence d'hydroxyde de sodium en tant que catalyseur. Ces polyglycols ont généralement un poids moléculaire compris entre environ 600 et 20000.

D'autres composés organiques préférés sont ceux qui ont des groupes mercapto, amino, carboxyle ou hydroxyle. Parmi ceux-ci, on cite plus particulièrement les composés polyhydroxy tels que les polyéther diols, les polyester diols, les polyacétal diols, les polyamide diols, les polyester polyamide diols, les poly(alkylène éther) diols, les polythioéther diols et les polycarbonate diols.

Les polyéther diols préférés sont, par exemple, les produits de condensation d'oxyde d'éthylène, d'oxyde de propylène ou de tétrahydrofurane, leurs produits de copolymérisation ou de condensation, greffés ou blocs, tels que les mélanges de condensats d'oxyde d'éthylène et de propylène, et les produits de polymérisation d'oléfines, sous haute pression, avec les condensats doxyde d'alkylène. Des polyéthers appropriés sont par exemple préparés par condensation d'oxydes d'alkylène et d'alcools polyhydriques, tels que l'éthylène glycol, le 1,2-propylène glycol et le 1,4-butanediol.

Les polyester diols, polyester amides, polyamide diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylène glycol, le 1,2-propylène glycol, le 1,4-butanediol, le néopentyl glycol et l'hexane diol. On peut aussi utiliser des aminoalcools, par exemple l'éthanolamine. Des diamines appropriées pour préparer les amides polyesters sont l'éthylène diamine et l'hexaméthylène diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycols soit seuls ou en combinaison avec d'autres glycols tels que l'éthylène glycol, le 1,2-propylène glycol ou avec d'autres composés polyhydroxylés. Les composés polyhydroxylés contenant déjà des groupements urée ou uréthanne, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester diol formé par la réaction d'au moins un (di)-polyol (1ₐ) et d'au moins un acide (1_{b}). Le (di)- polyol (1ₐ) est en particulier choisi dans le groupe comprenant le néopentylglycol, le butanediol-1,4, l'hexanediol, l'éthylèneglycol, le diéthylène glycol, le propylèneglycol, le butylèneglycol, le néopentylglycol et (di)-polyéthylèneglycol. L'acide (1_{b}) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylol propanoïque (DMPA) ou un acide carboxylique 2,2-hydroxyméthyl. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés conformément à l'invention sont ceux choisis dans le groupe comprenant le 2,2-di-(hydroxyméthyl) acide acétique, le 2,2-dihydroxyméthyl acide propionique, le 2,2-dihydroxyméthyl acide butyrique, l'acide 2,2-dihydroxyméthyl acide pentanoïque.

Le di- ou polyisocyanate (3) peut être choisi en particulier dans le groupe comprenant l'hexaméthylène diisocyanate, l'isophorondiisocyanate (IDPI), le toluylendiisocyanate, le diphénylméthane 4,4'-diisocyanate (DPMD) et le dicyclohexylméthane 4,4'-diisocyanate (DCMD), le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate, le 2,2'-dichloro-4,4'-diisocyanato diphénylméthane, le 2,4-dibromo-1,5-diisocyanato naphtalène, le 1,4-diisocyanate butane, l'hexane-1,6-diisocyanate, le cyclohexane-1,4-diisocyanate.

Le polycondensat peut être formé à l'aide d'un composé supplémentaire (4) servant en général à allonger la chaîne du polycondensat. Ces composés (4) peuvent être choisis dans la groupe comprenant notamment les glycols saturés
ou insaturés tel que l'éthylène glycol, le diéthylène glycol, le néopentylglycol, le triéthylène glycol, les aminoalcools tels que l'éthanolamine, la propanolamine, la butanolamine, les amines primaires hétérocyclique, aromatique, cycloaliphatique, et aliphatique, les diamines, les acides carboxylique tels que les acides carboxyliques aliphatique, aromatique, hétérocyclique comme l'acide oxalique, succinique, glutarique, adipique, sébacique, téréphtalique, les acides aminocarboxyliques. Les composés (4) préférés sont les diols aliphatiques.

Les polycondensats conformes à l'invention peuvent également être formés à partir de composés supplémentaires (5) ayant un squelette siliconé tels que les polysiloxanes, les polyalkylsiloxanes ou les polyarylsiloxanes notamment les polyéthylsiloxanes, les polyméthylsiloxanes et les polyphényisiloxanes, comportant éventuellement des chaînes hydrocarbonées greffées sur les atomes de silicium.

Selon un mode de réalisation avantageux des compositions conformes à l'invention, les séquences de polyuréthanne et/ou polyurée du polymère présentent un motif répétitif de base répondant à la formule générale 1 ci-après:

-X-B-X-CO-NH-R-NH-CO- (I)

dans laquelle :
- X représente O et/ou NH,
- B est un radical hydrocarboné bivalent, ce radical étant substitué ou non, et
- R est un radical divalent choisi parmi les radicaux alkylène de type aromatique, aliphatique en C₁ à C₂₀, cycloaliphatique en C₁ à C₂₀, ces radicaux étant substitués ou non.

De préférence, le radical B est un radical en C₁ à C₃₀ et est porteur d'un groupement présentant une ou des fonction(s) carboxylique(s) et/ou une ou des fonctions sulfoniques, lesdites fonctions carboxyliques et/ou sulfoniques étant sous forme libre ou bien neutralisées partiellement ou totalement par une base minérale ou organique.

Le radical R est avantageusement choisi parmi les radicaux répondant aux formules suivantes: 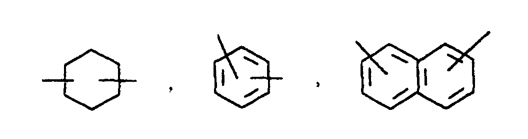 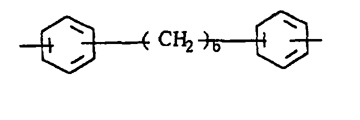 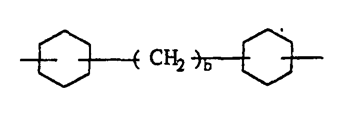 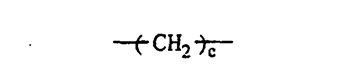 dans lesquelles b est un nombre entier compris entre 0 et 3, et c un nombre entier compris entre 1 et 20, de préférence compris entre 2 et 12.

En particulier, le radical R est choisi parmi les radicaux hexaméthylène, 4,4'-biphénylèneméthane, 2,4- et/ou 2,6-tolylène, 1,5-naphtylène, p-phénylène, méthylène- 4,4bis - cyclohéxyle et le radical divalent dérivé de l'isophorone.

Le polycondensat mis en oeuvre conformément à l'invention comprenant au moins une séquence polyuréthanne et/ou polyurée peut avantageusement comprendre en outre au moins une séquence polysiloxane dont le motif répétitif de base répond par exemple à la formule générale II ci-après:

-X-P-X-CO-NH-R-NH-CO- (II)

dans laquelle :
- P est un segment polysiloxanique,
- X représente O et/ou NH, et
- R est un radical divalent choisi parmi les radicaux alkylènes de type aromatique,. aliphatique en C₁ à C₂₀, cycloaliphatique en C₁ à C₂₀, ces radicaux étant substitués ou non.

Avantageusement, le segment polysiloxanique P répond à la formule générale III ci-après: 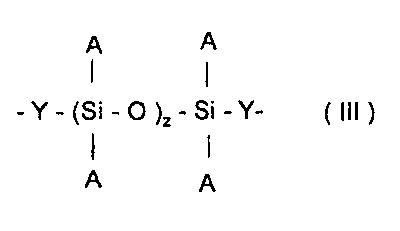 dans laquelle:
- les radicaux A, qui peuvent être identiques ou différents, sont choisis parmi d'une part les radicaux hydrocarbonés monovalents en C₁ à C₂₀ exempts ou substantiellement exempts d'insaturation éthylénique et, d'autre part, les radicaux aromatiques,
- Y représente un radical hydrocarboné bivalent, et
- z représente un nombre entier, choisi de telle sorte que le poids moléculaire moyen du segment polysiloxane soit compris entre 300 et 10 000.

En général, le radical bivalent Y est choisi parmi les radicaux alkylène de formule -(CH₂)ₐ- , dans laquelle a représente un nombre entier pouvant être compris entre 1 et 10.

Les radicaux A peuvent être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyle, en particulier le radical cyclohexyle, les radicaux aryle, notamment phényle et naphtyle, les radicaux arylalkyle, notamment benzyle et phényléthyle, ainsi que les radicaux tolyle et xylyle.

La composition conforme à l'invention comprend, en proportion relative en poids par rapport au poids total de la composition, entre 0,1 et 20 % du polycondensat comprenant au moins une séquence polyuréthanne et/ou polyurée, plus avantageusement entre 1 et 15 %, et plus avantageusement encore entre 2 et 8 % de ce polycondensat.

On utilise entre 7,5 et 70 % du solvant organique, plus avantageusement entre 10 et 50 %, et plus avantageusement encore entre 10 et 25 % en proportion relative en poids par rapport au poids total de la composition.

Conformément à l'invention, le solvant organique est notamment choisi dans le groupe comprenant les alcools inférieurs en C₁ à C₄ tels que l'éthanol, l'isopropanol, l'acétone, la méthyléthylcétone, l'acétate de méthyle, l'acétate de butyle, l'acétate d'éthyle, le diméthoxyéthane, le diéthoxyéthane et leurs mélanges. De manière préférentielle, on utilise l'éthanol.

Selon un mode de réalisation avantageux de la composition conforme à l'invention, elle comprend en proportion relative en poids par rapport au poids total de la composition, entre 25 et 60 % du gaz propulseur, et encore plus avantageusement entre 30 et 50 %.

Conformément à l'invention, on utilise, de préférence, comme gaz propulseur, un gaz soluble ou non dans la composition tel que le diméthyl éther, les hydrocarbures fluorés ou non, les gaz liquéfiés usuels ou un mélange de ces gaz propulseurs. Encore plus avantageusement, on utilise le diméthyléther.

La proportion relative en poids, par rapport au poids total de la composition, en polyol ou en mélange de polyols est comprise entre 0,01 et 20 %, plus avantageusement entre 0,01 et 10 %, et plus avantageusement encore entre 0,05 et 5 %.

Les polyols utilisés selon l'invention peuvent être choisis notamment parmi les polyols en C₂ à C₁₂ ainsi que les polyalkylèneglycols tels que plus particulièrement les polyéthylèneglycols et les polypropylèneglycols.

De préférence, on utilise comme polyol, un dérivé d'alcane polyhydroxylé en C₂ à C₈. Avantageusement, on choisit un composé en C₃ à C₅, et plus particulièrement encore la glycérine, le propylène glycol ou le 1,3- propanediol.

Les compositions conformes à l'invention peuvent par ailleurs contenir des additifs cosmétiques conventionnels choisis notamment parmi les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés, les silicones volatiles ou non, les protéines et les vitamines.

On porte un intérêt tout particulier à la réalisation de compositions cosmétiques conformes à l'invention pour lesquelles la quantité de composés organiques volatiles rejetés est de plus en plus faible.

En particulier, il peut être avantageux d'associer au polyuréthanne de l'invention au moins un autre polymère fixant anionique, cationique, non ionique ou amphotère.

L'invention pourra être mieux comprise à l'aide de l'exemple non limitatif qui suit et qui constitue un mode de réalisation avantageux des compositions conformes à l'invention.

### Exemple:

On réalise la composition capillaire conforme à l'invention ci-après.
- Polycondensat polyester acide lactique/ éthylène glycol P(MIS-EG) - acide diméthylol propanoïque (DMPA)- isophoronediisocyanate 4 g
- Glycérol 0,07 g
- Ethanol 15 g
- Diméthyléther 35 g -2-amino-2 méthyl-1-propanol qs neutralisation
- eau déminéralisée qsp 100 g

## Revendications

1. Composition capillaire destinée à être appliquée à partir d'un dispositif aérosol comprenant, dans un milieu cosmétiquement acceptable, en proportion relative en poids par rapport au poids total de la composition:
(i) 0,1 à 20 % d'un polycondensat comprenant au moins une séquence po lyuréthanne et/ou polyurée;
(ii) 7,5 à 70% d'un solvant organique;
(iii) 15 à 85 % d'un gaz propulseur,
**caractérisée par le fait que** la composition comprend en outre de 0,01 à 20 % d'au moins un polyol ou d'un mélange de ceux-ci, le rapport en poids du gaz propulseur au solvant organique étant supérieur ou égal à 1,75.

2. Composition selon la revendication 1, **caractérisée par le fait que** le polycondensat est formé par un arrangement de blocs obtenu à partir de:
(1) au moins un composé qui contient deux ou plus de deux atomes d'hydro gène actifs par molécule;
(2) au moins un diol ou un mélange de diols contenant des radicaux acides ou leurs sels;
(3) au moins un di- ou polyisocyanate.

3. Composition selon la revendication 2, **caractérisée par le fait que** les composés (1) sont choisis dans le groupe comprenant les diols, les diamines, les polyesterols, les polyétherols ou leur mélange.

4. Composition selon la revendication 2, **caractérisée par le fait que** le composé (2) est un acide carboxylique 2,2-hydroxyméthyl.

5. Composition selon la revendication 2, **caractérisée par le fait que** le composé (3) est choisi dans la groupe comprenant l'hexaméthylène diisocyanate, l'isophorondiisocyanate, le toluylendiisocyanate, le diphénylméthane 4,4'-diisocyanate, le dicyclohexylméthane 4,4'-diisocyanate, le méthylène-di-p-phényl diisocyanate, le méthylène-bis(4-cyclohexylisocyanate), l'isophorone diisocyanate, le toluène diisocyanate, le 1,5-naphtalène diisocyanate, le 4,4'-diphénylméthane diisocyanate, le 2,2'-diméthyl-4,4'-diphénylméthane diisocyanate, le 1,3-phénylène diisocyanate, le 1,4-phénylène diisocyanate, des mélanges de 2,4- et de 2,6- toluène diisocyanate. le 2,2'-dichloro-4,4'-diisocyanato diphénylméthane, le 2,4-dibromo-1,5-diisocyanato naphtalène, le 1,4-diisocyanate butane, l'hexane-1,6-diisocyanate et le cyclohexane-1,4-diisocyanate.

6. Composition selon la revendication 2, **caractérisée par le fait que** le polycondensat est formé à partir d'au moins un composé supplémentaire ayant un squelette siliconé et choisi dans le groupe comprenant les polysiloxanes, les polyalkylsiloxanes ou les polyarylsiloxanes.

7. Composition selon la revendication 6, **caractérisée par le fait que** les polyarylsiloxanes sont les polyéthylsiloxanes, les polyméthylsiloxanes et les polyphénylsiloxanes, comportant éventuellement des chaînes hydrocarbonées greffées sur les atomes de silicium.

8. Composition selon la revendication 1, **caractérisée par le fait que** les séquences de polyuréthanne et/ou polyurée du polycondensat présentent un motif répétitif de base répondant à la formule générale I' ci-après:
-X'-B-X'-CO-NH-R-NH-CO- (I')
dans laquelle :
- X' représente O et/ou NH,
- B est un radical hydrocarboné bivalent, ce radical étant substitué ou non, et
- R est un radical divalent choisi parmi les radicaux alkylène de type aromatique, aliphatique en C₁ à C₂₀, cycloaliphatique en C₁ à C₂₀, ces radicaux étant substitués ou non.

9. Composition selon la revendication 8, **caractérisée par le fait que** B est un radical hydrocarboné bivalent en C₁ à C₃₀.

10. Composition selon la revendication 8, **caractérisée par le fait que** le radical R est choisi dans le groupe comprenant les radicaux hexaméthylène, 4,4'-biphénylèneméthane, 2,4- et/ou 2,6-tolylène, 1,5-naphtylène, p-phénylène, méthylène- 4,4bis - cyclohéxyle et le radical divalent dérivé de l'isophorone.

11. Composition selon la revendication 1, **caractérisée par le fait que** le polycondensat présente un motif répétitif de base répondant à la formule (II'):
-X'-P-X'-CO-NH-R-NH-CO- (II')
dans laquelle :
- P est un segment polysiloxanique,
- X' représente O et/ou NH, et
- R est un radical divalent choisi parmi les radicaux alkylène de type aromatique, aliphatique en C₁ à C₂₀, cycloaliphatique en C₁ à C₂₀, ces radicaux étant substitués ou non.

12. Composition selon l'une quelconque des revendications précédentes, **caractérisée par le fait que** la composition comprend, en proportion relative en poids, entre 1 et 15 % du polycondensat.

13. Composition selon la revendication précédente12, **caractérisée par le fait que** la composition comprend, en proportion relative en poids, entre 2 et 8 % du polycondensat.

14. Composition selon l'une quelconque des revendications précédentes, **caractérisée par le fait que** la composition comprend entre 10 et 50 % du solvant organique.

15. Composition selon l'une quelconque des revendications précédentes, **caractérisée par le fait que** la composition comprend entre 10 et 25 % du solvant organique.

16. Composition selon l'une quelconque des revendications précédentes, **caractérisée par le fait que** le gaz propulseur est présent à une concentration relative en poids comprise entre 25 et 60 %.

17. Composition selon l'une quelconque des revendications précédentes, **caractérisée par le fait que** le gaz propulseur est présent à une concentration relative en poids comprise entre 30 et 50 %.

18. Composition selon l'une quelconque des revendications précédentes, **caractérisée par le fait que** la proportion relative en poids, par rapport au poids total de la composition, en polyol ou en mélange de polyols est comprise entre 0,01 et 10 %.

19. Composition selon l'une quelconque des revendications précédentes, **caractérisée par le fait que** la proportion relative en poids, par rapport au poids total de la composition, en polyol ou en mélange de polyols est comprise entre 0,05 et 5 %.

20. Composition selon l'une quelconque des revendications précédentes, **caractérisée par le fait que** le polyol est choisi dans le groupe comprenant les composés en C₂ à C₁₄ de type hydrocarbure aliphatique linéaire, ramifié ou cyclique, saturé ou insaturé, portant au moins deux fonctions hydroxyles sur la chaîne alkyle et les polymères de type polyéther de ces composés alkyles polyhydroxylés.

21. Composition selon la revendication 20, **caractérisée par le fait que** le polyol est un dérivé d'alcane polyhydroxylé en C₂ à C₁₂.

22. Composition selon la revendication 21, **caractérisée par le fait que** le polyol est un dérivé d'alcane polyhydroxylé en C₂ à C_{8.}

23. Composition selon la revendication 21, **caractérisée par le fait que** le polyol est un dérivé d'alcane polyhydroxylé en C₃ à C_{5.}

24. Composition selon l'une quelconque des revendications précédentes, **caractérisée par le fait qu'**elle contient en outre des additifs cosmétiques conventionnels choisis dans le groupe comprenant les corps gras, les agents épaississants, les adoucissants, les agents anti-mousse, les agents hydratants, les agents antiperspirants, les agents alcalinisants, les colorants, les pigments, les parfums, les conservateurs, les tensioactifs, les polymères hydrocarbonés, les silicones volatiles ou non, notamment les silicones anioniques, les polyols, les protéines et les vitamines.

25. Composition selon l'une quelconque des revendications précédentes, **caractérisée par le fait que** la composition contient en outre au moins un polymère fixant additionnel choisi dans le groupe comprenant les polymères fixants non ioniques, cationiques, anioniques et amphotères.

26. Dispositif aérosol constitué par un récipient contenant une composition aérosol selon l'une quelconque des revendications précédentes dans un récipient approprié, ainsi qu'un moyen de distribution de la composition.

27. Procédé pour la mise en forme ou le maintien de la coiffure, **caractérisé par le fait qu'**il comprend la mise en oeuvre d'une composition conforme à l'une quelconque des revendications 1 à 24.

28. Utilisation d'une composition selon l'une quelconque des revendications 1 à 24 pour la fabrication de produits capillaires, en vue d'obtenir un maintien ou une mise en forme de la coiffure.

## Patentansprüche

1. Zusammensetzung für die Haarbehandlung, die aus einer Aerosolvorrichtung aufgebracht werden soll und die in einem kosmetisch akzeptablen Medium in relativen Gewichtsmengen, bezogen auf das Gesamtgewicht der Zusammensetzung, enthält:
(i) 0,1 bis 20 % Polykondensat, das mindestens eine Polyurethansequenz und/oder Polyharnstoffsequenz enthält;
(ii) 7,5 bis 70 % organisches Lösungsmittel; und
(iii) 15 bis 85 % Treibmittel,
**dadurch gekennzeichnet, dass** die Zusammensetzung ferner 0,01 bis 20 % mindestens eines Polyols oder eines Gemisches von Polyolen enthält, wobei das Gewichtsverhältnis von Treibgas und organischem Lösungsmittel mindestens 1,75 beträgt.

2. Zusammensetzung nach Anspruch 1, **dadurch gekennzeichnet, dass** das Polykondensat aus einer Anordnung von Blöcken besteht, die erhalten wird ausgehend von:
(1) mindestens einer Verbindung, die zwei oder mehrere aktive Wasserstoffe pro Molekül enthält;
(2) mindestens einem Diol oder einem Gemisch von Diolen, die saure Gruppen oder deren Salze enthalten; und
(3) mindestens einem Di- oder Polyisocyanat.

3. Zusammensetzung nach Anspruch 2, **dadurch gekennzeichnet, dass** die Verbindungen (1) unter den Diolen, Diaminen, Polyesterolen, Polyetherolen oder deren Gemischen ausgewählt sind.

4. Zusammensetzung nach Anspruch 2, **dadurch gekennzeichnet, dass** die Verbindung (2) eine 2,2-Hydroxymethylcarbonsäure ist.

5. Zusammensetzung nach Anspruch 2, **dadurch gekennzeichnet, dass** die Verbindung (3) unter Hexamethylendiisocyanat, Isophorondiisocyanat, Toluylendiisocyanat, Diphenylmethan-4,4'-diisocyanat, Dicyclohexylmethan-4,4'-diisocyanat, Methylen-di-p-phenyldiisocyanat, Methylen-bis(4-cyclohexylisocyanat), Isophorondiisocyanat, Toluoldiisocyanat, 1,5-Naphthalindiisocyanat, 4,4'-Diphenylmethandiisocyanat, 2,2'-Dimethyl-4,4'diphenylmethandiisocyanat, 1,3-Phenylendiisocyanat, 1,4-Phenylendiisocyanat, Gemischen von 2,4- und 2,6-Toluoldiisocyanat, 2,2'-Dichlor-4,4'-diisocyanato-diphenylmethan, 2,4-Dibrom-1,5-diisocyanato-naphthalin, 1,4-Diisocyanatobutan, Hexan-1,6-diisocyanat und Cyclohexan-1,4-diisocyanat ausgewählt ist.

6. Zusammensetzung nach Anspruch 2, **dadurch gekennzeichnet, dass** das Polykondensat ausgehend von mindestens einer zusätzlichen Verbindung gebildet wird, welche ein Silicongerüst aufweist und unter den Polysiloxanen, Polyalkylsiloxanen und Polyarylsiloxanen ausgewählt ist.

7. Zusammensetzung nach Anspruch 6, **dadurch gekennzeichnet, dass** die Polyarylsiloxane Polyethylsiloxane, Polymethylsiloxane und Polyphenylsiloxane sind, die gegebenenfalls auf die Siliciumatome gepfropfte Kohlenwasserstoffketten enthalten.

8. Zusammensetzung nach Anspruch 1, **dadurch gekennzeichnet, dass** die Polyurethansequenzen und/oder Polyharnstoffsequenzen des Polykondensat eine wiederkehrende Grundeinheit der folgenden allgemeinen Formel (I') besitzen:
-X'-B-X'-CO-NH-R-NH-CO- (I'),
worin bedeuten:
- X' O und/oder NH,
- B eine zweiwertige Kohlenwasserstoffgruppe, wobei diese Gruppe unsubstituiert oder substituiert vorliegt, und
- R eine zweiwertige Gruppe, die unter den Alkylengruppen vom aromatischen Typ, vom aliphatischen Typ mit 1 bis 20 Kohlenstoffatomen und vom cycloaliphatischen Typ mit 1 bis 20 Kohlenstoffatomen ausgewählt ist, wobei diese Gruppen substituiert oder unsubstituiert sein können.

9. Zusammensetzung nach Anspruch 8, **dadurch gekennzeichnet, dass** die Gruppe B eine zweiwertige C₁₋₃₀-Kohlenwasserstoffgruppe ist.

10. Zusammensetzung nach Anspruch 8, **dadurch gekennzeichnet, dass** die Gruppe R unter den Gruppen Hexamethylen, 4,4'-Biphenylenmethan, 2,4- und/oder 2,6-Toluylen, 1,5-Naphthylen, p-Phenylen, Methylen-4,4-bis-cyclohexyl und der von Isophoron abgeleiteten zweiwertigen Gruppe ausgewählt ist.

11. Zusammensetzung nach Anspruch 1, **dadurch gekennzeichnet, dass** das Polykondensat eine wiederkehrende Grundeinheit der folgenden Formel (II') enthält:
-X'-P-X'-CO-NH-R-NH-CO- (II'),
worin bedeuten:
- P eine Polysiloxansegment,
- X' O und/ oder NH,
- R eine zweiwertige Gruppe, die unter den Alkylengruppen vom aromatischen Typ, vom aliphatischen Typ mit 1 bis 20 Kohlenstoffatomen und vom cycloaliphatischen Typ mit 1 bis 20 Kohlenstoffatomen ausgewählt ist, wobei diese Gruppen substituiert oder unsubstituiert sein können.

12. Zusammensetzung nach einem der vorhergehenden Ansprüche, **dadurch gekennzeichnet, dass** die Zusammensetzung 1 bis 15 Gew.-% Polykondensat enthält.

13. Zusammensetzung nach Anspruch 12, **dadurch gekennzeichnet, dass** die Zusammensetzung das Polykondensat in einer relativen Gewichtsmenge von 2 bis 8 % enthält.

14. Zusammensetzung nach einem der vorhergehenden Ansprüche, **dadurch gekennzeichnet, dass** die Zusammensetzung 10 bis 50 % organisches Lösungsmittel enthält.

15. Zusammensetzung nach einem der vorhergehenden Ansprüche, **dadurch gekennzeichnet, dass** die Zusammensetzung 10 bis 25 % organisches Lösungsmittel enthält.

16. Zusammensetzung nach einem der vorhergehenden Ansprüche, **dadurch gekennzeichnet, dass** das Treibgas in einer auf das Gewicht bezogenen relativen Konzentration von 25 bis 60 % vorliegt.

17. Zusammensetzung nach einem der vorhergehenden Ansprüche, **dadurch gekennzeichnet, dass** das Treibgas in einer auf das Gewicht bezogenen relativen Konzentration von 30 bis 50 % vorliegt.

18. Zusammensetzung nach einem der vorhergehenden Ansprüche, **dadurch gekennzeichnet, dass** die relative Gewichtsmenge, bezogen auf das Gesamtgewicht der Zusammensetzung, des Polyols oder des Gemisches von Polyolen im Bereich von 0,1 bis 10 % liegt.

19. Zusammensetzung nach einem der vorhergehenden Ansprüche, **dadurch gekennzeichnet, dass** die relative Gewichtsmenge, bezogen auf das Gesamtgewicht der Zusammensetzung, des Polyols oder des Gemisches von Polyolen im Bereich von 0,05 bis 5 % liegt.

20. Zusammensetzung nach einem der vorhergehenden Ansprüche, **dadurch gekennzeichnet, dass** das Polyol unter den Verbindungen vom Typ der aliphatischen, geradkettigen, verzweigten oder cyclischen, gesättigten oder ungesättigten Kohlenwasserstoffe mit 2 bis 14 Kohlenstoffatomen, die mindestens zwei Hydroxygruppen an der Alkylkette aufweisen, und den Polymeren dieser mehrfach hydroxylierten Alkylverbindungen vom Polyethertyp ausgewählt ist.

21. Zusammensetzung nach Anspruch 20, **dadurch gekennzeichnet, dass** das Polyol ein mehrfach hydroxyliertes C₂₋₁₂-Alkanderivat ist.

22. Zusammensetzung nach Anspruch 21, **dadurch gekennzeichnet, dass** das Polyol ein mehrfach hydroxyliertes C₂₋₈-Alkanderivat ist.

23. Zusammensetzung nach Anspruch 21, **dadurch gekennzeichnet, dass** das Polyol ein mehrfach hydroxyliertes C₃₋₅-Alkanderivat ist.

24. Zusammensetzung nach einem der vorhergehenden Ansprüche, **dadurch gekennzeichnet, dass** sie ferner herkömmliche kosmetische Zusatzstoffe enthält, die unter den Fettsubstanzen, Verdickungsmitteln, beruhigenden Stoffen, Schaumverhütungsmitteln, Hydratisierungsmitteln, Antiperspirantien, Alkalisierungsmitteln, Farbmitteln, Pigmenten, Parfums, Konservierungsmitteln, grenzflächenaktiven Stoffen, Polymeren auf Kohlenwasserstoffbasis, flüchtigen oder nicht flüchtigen Siliconen und insbesondere anionischen Siliconen, Polyolen, Proteinen und Vitaminen ausgewählt sind.

25. Zusammensetzung nach einem der vorhergehenden Ansprüche, **dadurch gekennzeichnet, dass** die Zusammensetzung ferner ein zusätzliches fixierendes Polymer enthält, das unter den nichtionischen, kationischen, anionischen und amphoteren fixierenden Polymeren ausgewählt ist.

26. Aerosolvorrichtung, die aus einem Behälter, der eine Aerosolzusammensetzung nach einem der vorhergehenden Ansprüche in einem geeigneten Behälter enthält, sowie einem Mittel zur Verteilung der Zusammensetzung besteht.

27. Verfahren zur Formgebung oder Festigung der Frisur, **dadurch gekennzeichnet, dass** es die Verwendung einer Zusammensetzung nach einem der Ansprüche 1 bis 24 umfasst.

28. Verwendung einer Zusammensetzung nach einem der Ansprüche 1 bis 24 zur Herstellung von Produkten für die Haarbehandlung zur Festigung oder Formgebung der Frisur.

## Claims

1. Hair composition intended to be applied using an aerosol device comprising, in a cosmetically acceptable medium, in a relative proportion by weight relative to the total weight of the composition:
(i) 0.1 to 20% of a polycondensate comprising at least one polyurethane and/or polyurea sequence,
(ii) 7.5 to 70% of an organic solvent,
(iii) 15 to 85% of a propellent gas,
**characterized in that** the composition also comprises from 0.01 to 20% of at least one polyol or a mixture thereof, the weight ratio of the propellent gas to the organic solvent being greater than or equal to 1.75.

2. Composition according to Claim 1,
**characterized in that** the polycondensate is formed by an arrangement of blocks obtained using:
(1) at least one compound which contains two or more than two active hydrogen atoms per molecule;
(2) at least one diol or a mixture of diols containing acid radicals or their salts;
(3) at least one di- or polyisocyanate.

3. Composition according to Claim 2,
**characterized in that** the compounds (1) are chosen from the group consisting of diols, diamines, polyesterols and polyetherols, or a mixture thereof.

4. Composition according to Claim 2,
**characterized in that** the compound (2) is a 2,2-hydroxymethylcarboxylic acid.

5. Composition according to Claim 2,
**characterized in that** the compound (3) is chosen from the group consisting of hexamethylene diisocyanate, isophorone diisocyanate, toluylene diisocyanate, diphenylmethane 4,4'-diisocyanate, dicyclohexylmethane 4,4'-diisocyanate, methylenedi(p-phenyl) diisocyanate, methylenebis(4-cyclohexyl isocyanate), isophorone diisocyanate, toluene diisocyanate, 1,5-naphthalene diisocyanate, 4,4'-diphenylmethane diisocyanate, 2,2'-dimethyl-4,4'-diphenylmethane diisocyanate, 1,3-phenylene diisocyanate, 1,4-phenylene diisocyanate, mixtures of 2,4- and 2,6-toluene diisocyanate, 2,2'-dichloro-4,4'-diisocyanatodiphenylmethane, 2,4-dibromo-1,5-diisocyanatonaphthalene, butane 1,4-diisocyanate, 1,6-hexane diisocyanate and 1,4-cyclohexane diisocyanate.

6. Composition according to Claim 2,
**characterized in that** the polycondensate is formed from at least one additional compound having a silicone skeleton and chosen from the group consisting of polysiloxanes, polyalkylsiloxanes or polyarylsiloxanes.

7. Composition according to Claim 6,
**characterized in that** the polyarylsiloxanes are polyethylsiloxanes, polymethylsiloxanes and polyphenylsiloxanes, optionally containing hydrocarbon-based chains grafted onto the silicon atoms.

8. Composition according to Claim 1,
**characterized in that** the polyurethane and/or polyurea sequences of the polycondensate have a repeating base unit corresponding to the general formula I' below:
-X'-B-X'-CO-NH-R-NH-CO- (I')
in which:
- X' represents O and/or NH,
- B is a divalent hydrocarbon-based radical, this radical being substituted or unsubstituted, and
- R is a divalent radical chosen from alkylene radicals of aromatic type, C₁ to C₂₀ aliphatic radicals or C₁ to C₂₀ cycloaliphatic radicals, these radicals being substituted or unsubstituted.

9. Composition according to Claim 8,
**characterized in that** B is a C₁ to C₃₀ divalent hydrocarbon-based radical.

10. Composition according to Claim 8,
**characterized in that** the radical R is chosen from the group consisting of hexamethylene, 4,4'-biphenylenemethane, 2,4- and/or 2,6-tolylene, 1,5-naphthylene, p-phenylene and methylene-4,4-bis-cyclohexyl radicals and the divalent radical derived from isophorone.

11. Composition according to Claim 1,
**characterized in that** the polycondensate has a repeating base unit corresponding to formula (II'):
-X'-P-X'-CO-NH-R-NH-CO- (II')
in which:
- P is a polysiloxane segment,
- X' represents O and/or NH, and
- R is a divalent radical chosen from alkylene radicals of aromatic type, C₁ to C₂₀ aliphatic radicals and C₁ to C₂₀ cycloaliphatic radicals, these radicals being substituted or unsubstituted.

12. Composition according to any one of the preceding claims, **characterized in that** the composition comprises, in a relative proportion by weight, between 1 and 15% of the polycondensate.

13. Composition according to the preceding Claim 12, **characterized in that** the composition comprises, in a relative proportion by weight, between 2 and 8% of the polycondensate.

14. Composition according to any one of the preceding claims, **characterized in that** the composition comprises between 10 and 50% of the organic solvent.

15. Composition according to any one of the preceding claims, **characterized in that** the composition comprises between 10 and 25% of the organic solvent.

16. Composition according to any one of the preceding claims, **characterized in that** the propellent gas is present at a relative concentration by weight of between 25 and 60%.

17. Composition according to any one of the preceding claims, **characterized in that** the propellent gas is present at a relative concentration by weight of between 30 and 50%.

18. Composition according to any one of the preceding claims, **characterized in that** the relative proportion by weight, relative to the total weight of the composition, of polyol or of a mixture of polyols is between 0.01 and 10%.

19. Composition according to any one of the preceding claims, **characterized in that** the relative proportion by weight, relative to the total weight of the composition, of polyol or of a mixture of polyols is between 0.05 and 5%.

20. Composition according to any one of the preceding claims, **characterized in that** the polyol is chosen from the group comprising C₂ to C₁₄ compounds of linear, branched or cyclic, saturated or unsaturated aliphatic hydrocarbon type bearing at least two hydroxyl functions on the alkyl chain and polymers of polyether type of these polyhydroxyalkyl compounds.

21. Composition according to Claim 20,
**characterized in that** the polyol is a C₂ to C₁₂ polyhydroxyalkane derivative.

22. Composition according to Claim 21, **characterized in that** the polyol is a C₂ to C₈ polyhydroxyalkane derivative.

23. Composition according to Claim 21,
**characterized in that** the polyol is a C₃ to C₅ polyhydroxyalkane derivative.

24. Composition according to any one of the preceding claims, **characterized in that** it also contains conventional cosmetic additives chosen from the group consisting of fatty substances, thickeners, softeners, antifoaming agents, moisturizers, antiperspirants, basifying agents, dyes, pigments, fragrances, preserving agents, surfactants, hydrocarbon-based polymers, volatile or non-volatile silicones, in particular anionic silicones, polyols, proteins and vitamins.

25. Composition according to any one of the preceding claims, **characterized in that** the composition also contains at least one additional fixing polymer chosen from the group comprising nonionic, cationic, anionic and amphoteric fixing polymers.

26. Aerosol device consisting of a container containing an aerosol composition according to any one of the preceding claims, in an appropriate container, as well as a means for distributing the composition.

27. Process for shaping or maintaining the hairstyle, **characterized in that** it comprises the use of a composition in accordance with any one of Claims 1 to 24.

28. Use of a composition according to any one of Claims 1 to 24 for the manufacture of hair products, in order to maintain or shape the hairstyle.
